# EUROPEAN PATENT APPLICATION

(11) **EP 4 123 016 A1**
(43) Date of publication of application: **25.01.2023**
(21) Application number: 21772548.0
(22) Date of filing: 18.03.2021
(51) Int. Cl.: C12N 5/077, A61K 35/34, A61L 27/38, A61P 9/10

(54) **METHOD FOR PURIFYING CARDIOMYOCYTES**

(30) Priority: 19.03.2020 JP 2020050269; 28.08.2020 JP 2020145097
(71) Applicant: Orizuru Therapeutics, Inc., Fujisawa-shi Kanagawa 251-8555 (JP)
(72) Inventor: YOSHIDA, Yoshinori, Kyoto-shi, Kyoto 606-8501 (JP); TANAKA, Aya, Fujisawa-shi, Kanagawa 251-0012 (JP); WATANABE, Kozo, Fujisawa-shi, Kanagawa 251-0012 (JP); KONDO, Shigeru, Fujisawa-shi, Kanagawa 251-0012 (JP); NISHIMOTO, Tomoyuki, Fujisawa-shi, Kanagawa 251-0012 (JP)
(74) Representative: J A Kemp LLP
(86) International application number: PCT/JP2021/011232
(87) International publication number: WO 2021/187602

(57) **Abstract**

A method for producing a cell population containing cardiomyocytes, including (1) a step of bringing a histone deacetylase inhibitor into contact with a cell population containing cardiomyocytes and cells other than cardiomyocytes, the cell population being obtained by culturing pluripotent stem cells in a medium for cardiomyocyte differentiation, and (2) a step of culturing the cell population is provided by the present invention.

## Description

### [Technical Field]

The present invention relates to a production method and a purification method of cardiomyocytes. More particularly, the present invention relates to a production method and a purification method of cardiomyocytes by using a histone deacetylase inhibitor.

### (Background of the Invention)

Although the incidence of myocardial infarction has been decreasing in recent years, cardiac diseases including myocardial infarction still remain the leading cause of death worldwide. Heart transplantation is currently the only treatment for patients with severe heart failure, but the shortage of donors poses a problem for heart transplantation. Therefore, cell therapy using cardiomyocytes is attracting attention as a therapeutic method that improves heart disease. In addition, establishment of an in vitro drug efficacy evaluation test or drug safety test using cardiomyocytes is also attracting attention. Therefore, a stable supply of uniform cardiomyocytes that can be used for cell therapy and in vitro tests is required.

One of the methods for stably providing homogeneous cardiomyocytes is to induce the differentiation of cardiomyocytes from stem cells or cardiomyocyte progenitor cells, and various efforts have been made to establish an efficient method for inducing differentiation into cardiomyocytes. As such differentiation induction method, a method for promoting differentiation induction from pluripotent stem cells into cardiomyocytes by culturing pluripotent stem cells in a medium containing an EGFR inhibitor (Patent Literature 1), a method for inducing differentiation of induced pluripotent stem cells into cardiomyocytes and then maturing the cardiomyocytes by contacting the cardiomyocytes with a Neuregulin 1 antagonist or ErbB antagonist (Patent Literature 2), a method for promoting differentiation induction of undifferentiated progenitor cells such as myoblasts by contacting the undifferentiated progenitor cells with a deacetylase inhibitor (Patent Literature 3), a method for promoting differentiation induction of adult progenitor cells such as myocardial progenitor cells and the like by contacting the progenitor cells with a histone deacetylase (HDAC) inhibitor (Patent Literature 4), and the like have been reported. In addition, a method that does not undergo a process of differentiation induction from stem cells has also been reported. For example, Patent Literature 5 discloses a method for producing myocardial progenitor cells or cardiomyocytes from somatic cells such as fibroblasts and the like by direct reprogramming.

### [Citation List]

### [Patent Literature]

[PTL 1]
   WO 2014/136519
[PTL 2]
   US-A-2010/0183565
[PTL 3]
   WO 2003/033678
[PTL 4]
   WO 2009/073618
[PTL 5]
   WO 2015/038704

### [Summary of Invention]

### [Technical Problem]

An object of the present invention is to provide a method for producing a cell population containing high-purity cardiomyocytes by means different from the above-mentioned conventional methods. Another object of the present invention is to provide a method for purifying cardiomyocytes at high purity from a cell population containing cardiomyocytes.

### [Solution to Problem]

The present inventors have conducted intensive studies in an attempt to solve the above-mentioned problems and acquired an idea that the ratio of cardiomyocytes in the cell population may be increased, in other words, cardiomyocytes may be purified by adding a compound that suppresses the proliferation of cells other than cardiomyocytes or a compound that reduces the number of cells other than cardiomyocytes, to a cell population already containing cardiomyocytes, rather than promoting differentiation induction from undifferentiated cells into cardiomyocytes. Thus, compounds that markedly reduce the number of iPS cells but show less effect on cardiomyocytes were screened for by adding compound libraries to iPS cells and cardiomyocytes induced to differentiate from iPS cells. As a result, plural kinds of HDAC inhibitors were suggested as candidates for compounds having a cardiomyocyte purification action. Then, embryoid bodies containing cardiomyocytes were contacted with the candidate compounds, and it was found that cardiomyocytes can be successfully purified. The present inventors have conducted further studies based on these findings and completed the present invention.

Therefore, the present invention provides the following.
[1] A method for producing a cell population comprising cardiomyocytes, comprising
   (1) a step of bringing a histone deacetylase inhibitor into contact with a cell population containing cardiomyocytes and cells other than cardiomyocytes, the cell population being obtained by culturing pluripotent stem cells in a medium for cardiomyocyte differentiation, and
   (2) a step of culturing the cell population.
[2] The method of [1], wherein the cell population is contacted with the histone deacetylase inhibitor in the aforementioned step (1) on or after 7 days from the start of differentiation induction of pluripotent stem cells.
[3] The method of [1] or [2], wherein the aforementioned inhibitor is an inhibitor against class I histone deacetylase.
[4] The method of any of [1] to [3], wherein the aforementioned inhibitor is at least one member selected from the group consisting of FK228, Entinostat, Trichostatin A, and Panobinostat.
[5] The method of any of [1] to [4], wherein the aforementioned pluripotent stem cell is an induced pluripotent stem cell.
[6] A method for removing or reducing undifferentiated cells from a cell population comprising cardiomyocytes and contaminated with the undifferentiated cells, comprising a step of contacting a histone deacetylase inhibitor with the cell population.
[7] The method of [6], wherein the aforementioned undifferentiated cell is a pluripotent stem cell.
[8] A cell population comprising cardiomyocytes and obtained by the method of any of [1] to [7].
[9] An agent for cell transplantation therapy, comprising the cell population of [8].
[10] A method for purifying cardiomyocytes, comprising
   (1) a step of bringing a histone deacetylase inhibitor into contact with a cell population containing cardiomyocytes and cells other than cardiomyocytes, the cell population being obtained by culturing pluripotent stem cells in a medium for cardiomyocyte differentiation, and
   (2) a step of culturing the cell population.

### [Advantageous Effects of Invention]

According to the present invention, a cell population containing cardiomyocytes at a high purity is provided. Such cell population can be preferably used in cell transplantation therapy for cardiac diseases. In addition, a method for purifying cardiomyocytes at a high purity from a cell population containing the cardiomyocytes, and a method for reducing cells other than cardiomyocytes (i.e., non-cardiomyocytes) from a cell population containing cardiomyocytes are also provided.

### [Brief Description of Drawings]

[Fig. 1]

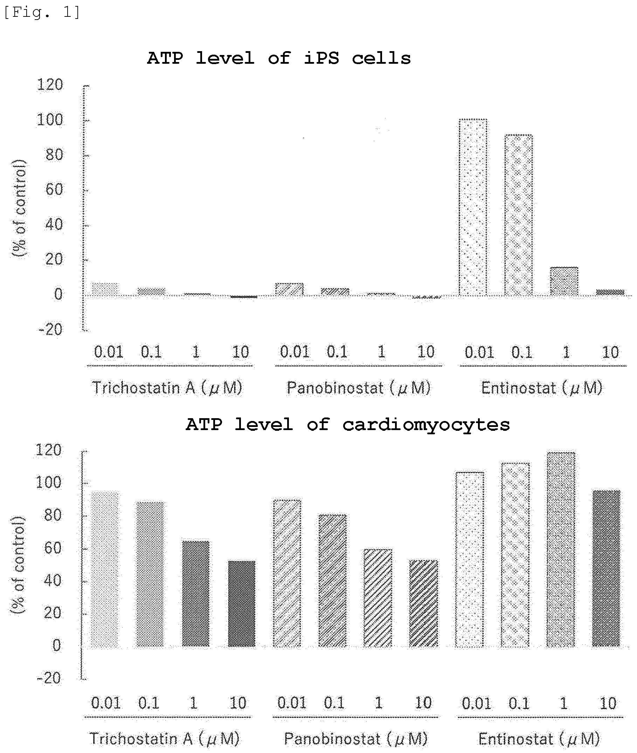

   Fig. 1 shows the intracellular ATP levels of undifferentiated iPS cells (cardiac muscle reporter iPS cell line) and purified cardiomyocytes derived from the said iPS cell and after treatment with a histone deacetylase inhibitor, in the compound screening of Experimental Example 1.
[Fig. 2]

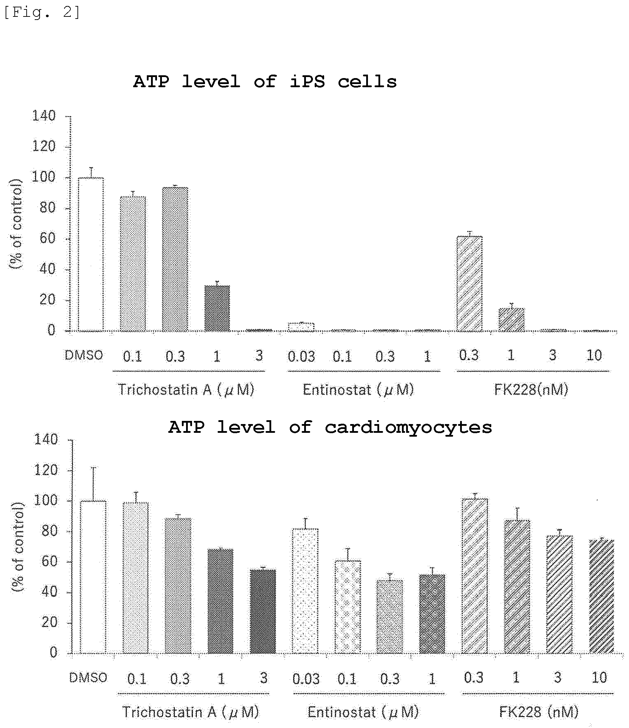

   Fig. 2 shows the intracellular ATP levels of iPS cells and cardiomyocytes derived from the said iPS cell and after treatment with a histone deacetylase inhibitor in Experimental Example 2.
[Fig. 3]

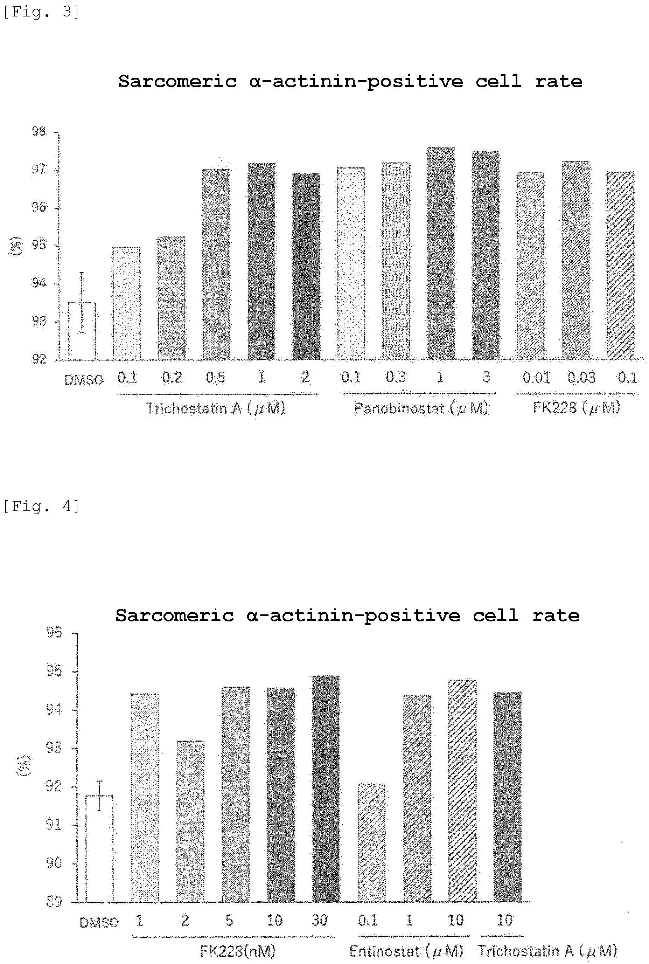

   Fig. 3 shows the sarcomere α-actinin-positive cell rate by flow cytometer analysis of CiRA clinical iPS cell-derived cardiomyocytes after treatment with a histone deacetylase inhibitor in Experimental Example 3.
[Fig. 4]
   Fig. 4 shows the sarcomere α-actinin-positive cell rate by flow cytometer analysis of iPS cell-derived cardiomyocytes after treatment with a histone deacetylase inhibitor in Experimental Example 4.
[Fig. 5]

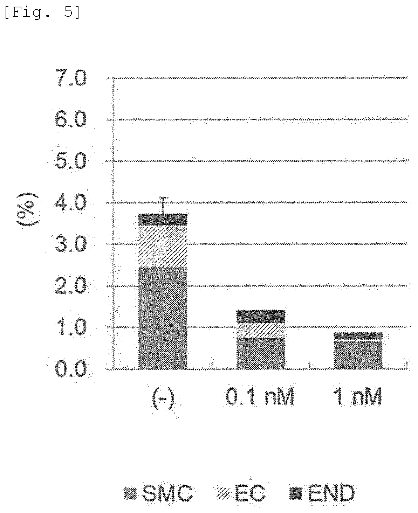

   Fig. 5 shows the non-cardiomyocyte rate after treatment with compound in Experimental Example 5 (END: endoderm lineage cells, SMC: smooth muscle-like cells, EC: endothelium-like cells).

### (Detailed Description of the Invention)

### 1. Production method of cell population containing cardiomyocytes

The present invention provides a method for producing a cell population containing cardiomyocytes (hereinafter also to be referred to as "the production method of the present invention"). The production method of the present invention includes (1) a step of bringing a histone deacetylase (HDAC) inhibitor into contact with a cell population containing cardiomyocytes and cells other than cardiomyocytes, and (2) a step of culturing the cell population.

In the present specification, the "cardiomyocytes" means a cell positive for at least one of sarcomeric α-actinin, cardiac troponin T (cTnT), and troponin I type 1, and is preferably a sarcomeric α-actinin-positive cell. Typically, it is a cardiac muscle cell having self-beating ability. The "cells other than cardiomyocytes" means a cell that is not cardiomyocyte, and specific examples thereof include smooth muscle cell, endothelial cell, stem cell (e.g., pluripotent stem cell), cardiac progenitor cell, and the like.

In the present specification, "positive" means that a protein or a gene is expressed in an amount detectable by at least any method known in the art. Protein can be detected by an immunological assay using an antibody, such as ELISA, immunostaining, or flow cytometry. In the case of a protein that is intracellularly expressed and does not appear on the cell surface (e.g., transcription factor or subunit thereof, and the like), a reporter protein is expressed together with the protein, and the target protein can be detected by detecting the reporter protein. Gene can be detected by, for example, a nucleic acid amplification method and/or a nucleic acid detection method such as RT-PCR, biochip (e.g., microarray), RNA seq, or the like. The expression of a protein or a gene can be determined by a general method. For example, when flow cytometry is utilized and the expression level is relatively high as compared with the expression level of the control group showing negative protein expression, the protein can be determined to be detectably expressed.

In the present specification, "negative" means that the expression level of the protein or a gene is less than the lower marginal by all or any of the above-mentioned known methods. The lower marginal of the expression of a protein or a gene may vary depending on each method, but can be determined by a general method.

In the present specification, the "cell population" means a population composed of two or more cells of the same type or different types. The "cell population" also means a mass of cells of the same type or different types. A cell population containing cardiomyocytes and cells other than cardiomyocytes to be used in the aforementioned step (1) can be produced by culturing pluripotent stem cells in a medium for cardiomyocyte differentiation. Therefore, the production method of the present invention may include, before the aforementioned step (1), step (0) of inducing differentiation of cardiomyocytes from pluripotent stem cells.

Examples of the pluripotent stem cell to be used in the present invention include induced pluripotent stem cell (iPS cell), embryonic stem cell (ES cell), embryonic stem cell derived from clone embryo obtained by nucleus transplantation (nuclear transfer Embryonic stem cell: ntES cell), multipotent germline stem cell (mGS cell), embryonic germ cell (EG cell), Muse cell (multi-lineage differentiating stress enduring cell). iPS cell is preferred (human iPS cell is more preferred). When the above-mentioned pluripotent stem cell is ES cell or any cell derived from human embryo, the cell may be a cell produced by destroying the embryo or a cell prepared without destroying the embryo. Preferably, it is a cell prepared without destroying the embryo. The above-mentioned pluripotent stem cell is preferably derived from a mammal (e.g., mouse, rat, hamster, guinea pig, dog, monkey, orangutan, chimpanzee, human), more preferably human. Therefore, the pluripotent stem cell to be used in the present invention is most preferably human iPS cell.

The "induced pluripotent stem cell (iPS cell)" refers to a cell obtained by reprogramming by introducing a specific factor (nuclear reprogramming factor) into a mammalian somatic cell or undifferentiated stem cell. At present, there are various types of "induced pluripotent stem cells (iPS cell)". In addition to the iPS cell established by Yamanaka et al. by introducing four factors of Oct3/4, Sox2, Klf4, and c-Myc into mouse fibroblasts (Takahashi K, Yamanaka S., Cell, (2006) 126: 663-676), human cell -derived iPS cells established by introducing the same four factors into human fibroblasts (Takahashi K, Yamanaka S., et al. Cell, (2007) 131: 861-872.), Nanog-iPS cell established by selecting Nanog expression as an index after introducing the above-mentioned four factors (Okita, K., Ichisaka, T., and Yamanaka, S. (2007). Nature 448, 313-317.), iPS cell prepared by a method that does not contain c-Myc (Nakagawa M, Yamanaka S., et al. Nature Biotechnology, (2008) 26, 101-106), and iPS cell established by introducing six factors by a virus-free method (Okita K et al. Nat. Methods 2011 May; 8(5):409-12, Okita K et al. Stem Cells. 31(3):458-66.) can also be used. In addition, induced pluripotent stem cell established by introducing four factors of OCT3/4, SOX2, NANOG, and LIN28 produced by Thomson et al. (Yu J., Thomson JA. et al., Science (2007) 318: 1917-1920.), induced pluripotent stem cell produced by Daley et al. (Park IH, Daley GQ. et al., Nature (2007) 451: 141-146), induced pluripotent stem cell produced by Sakurada et al. (JP-A-2008-307007), and the like can also be used.

In addition, any of the induced pluripotent stem cells known in the art which are described in all published papers (e.g., Shi Y., Ding S., et al., Cell Stem Cell, (2008) Vol3, Issue 5, 568-574; , Kim JB., Scholer HR., et al., Nature, (2008) 454, 646-650; Huangfu D., Melton, DA., et al., Nature Biotechnology, (2008) 26, No 7, 795-797) or patents (e.g., JP-A-2008-307007, JP-A-2008-283972, US2008-2336610, US2009-047263, WO2007-069666, WO2008-118220, WO2008-124133, WO2008-151058, WO2009-006930, WO2009-006997, WO2009-007852) can be used. As the induced pluripotent stem cell line, various iPS cell lines established by NIH, RIKEN, Kyoto University, and the like can be used. For example, in the case of human iPS cell line, HiPS-RIKEN-1A strain, HiPS-RIKEN-2A strain, HiPS-RIKEN-12A strain, and Nips-B2 strain of RIKEN, and 253G1 strain, 201B7 strain, 409B2 strain, 454E2 strain, 606A1 strain, 610B1 strain, 648A1 strain, and iPS cell stock for regenerative medicine of Kyoto University, and the like can be mentioned.

The "somatic cell" in the present specification means any animal cell excluding germ line cells and differentiated totipotent cells such as ovum, oocyte, ES cells and the like (preferably, cells of mammals inclusive of human). Somatic cell is not particularly limited and encompasses any of somatic cells of fetuses, somatic cells of neonates, and mature healthy or pathogenic somatic cells, and any of primary cultured cells, passage cells, and established lines of cells. Specific examples of the somatic cell include (1) tissue stem cells (somatic stem cells) such as neural stem cell, hematopoietic stem cell, mesenchymal stem cell, dental pulp stem cell, and the like, (2) tissue progenitor cell, (3) differentiated cells such as lymphocyte, epithelial cell, endothelial cell, myocyte, fibroblast (skin cell, etc.), hair cell, hepatocyte, gastric mucosal cell, enterocyte, splenocyte, pancreatic cell (pancreatic exocrine cell, etc.), brain cell, lung cell, renal cell, adipocyte, and the like, and the like.

ES cell is a pluripotent and self-replicating stem cell established from the inner cell mass of early embryo (e.g., blastocyst) of a mammal such as human, mice, or the like. ES cell was discovered in mouse in 1981 (M.J. Evans and M.H. Kaufman (1981), Nature 292:154-156), after which ES cell line was also established in primates such as human, monkey and the like (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cell can be established by removing the inner cell mass from the blastocyst of a fertilized egg of the target animal and culturing the inner cell mass on a fibroblast feeder. Methods for establishing and maintaining human and monkey ES cells are described in, for example, USP5,843,780; Thomson JA, et al. (1995), Proc Natl. Acad. Sci. USA. 92:7844-7848; Thomson JA, et al. (1998), Science. 282:1145-1147; Suemori H. et al. (2006), Biochem. Biophys. Res. Commun., 345:926-932; Ueno M. et al. (2006), Proc. Natl. Acad. Sci. USA, 103:9554-9559; Suemori H. et al. (2001), Dev. Dyn., 222:273-279; Kawasaki H. et al. (2002), Proc. Natl. Acad. Sci. USA, 99:1580-1585; Klimanskaya I. et al. (2006), Nature. 444:481-485, and the like. Alternatively, ES cell can be established using only single blastomere of an embryo in the cleavage stage before the blastocyst stage (Chung Y. et al. (2008), Cell Stem Cell 2: 113-117), or can also be established using embryo that has stopped developing (Zhang X. et al. (2006), Stem Cells 24: 2669-2676.). As for "ES cell ", various mouse ES cell lines established by inGenious targeting laboratory, RIKEN, and the like can be used for mouse ES cells. For human ES cell, various human ES cell lines established by the University of Wisconsin, NIH, RIKEN, Kyoto University, the National Center for Child Health and Development, Cellartis, and the like can be used. For example, as human ES cell lines, CHB-1 to CHB-12 strains, RUES1 strain, RUES2 strain, HUES1 to HUES28 strains, and the like distributed by ESI Bio, and H1 strain, H9 strain, and the like distributed by WiCell Research, and KhES-1 strain, KhES-2 strain, KhES-3 strain, KhES-4 strain, KhES-5 strain, SSES1 strain, SSES2 strain, SSES3 strain, and the like distributed by RIKEN can be utilized.

nt ES cell (nuclear transfer ES cell) is an ES cell derived from a cloned embryo prepared by a nuclear transplantation technique, and has almost the same property as the ES cell derived from a fertilized egg (Wakayama T. et al. (2001), Science, 292: 740-743; S. Wakayama et al. (2005), Biol. Reprod., 72: 932-936; J. Byrne et al. (2007), Nature, 450: 497-502). That is, an ES cell established from an inner cell mass of a blastocyst derived from a cloned embryo obtained by substituting the nucleus of an unfertilized egg with the nucleus of a somatic cell is an nt ES (nuclear transfer ES) cell. For production of an nt ES cell, a combination of the nuclear transplantation technique (Cibelli J.B. et al. (1998), Nature Biotechnol., 16: 642-646) and the ES cell production technique (mentioned above) is used (Kiyoka Wakayama et al., (2008), Experimental Medicine, Vol. 26, No. 5 (Suppl.), pp. 47 to 52). In nuclear transplantation, reprogramming can be performed by injecting the nucleus of a somatic cell to an enucleated unfertilized egg of a mammal, and culturing for a few hours.

mGS cell is a pluripotent stem cell derived from the testis, which becomes the origin for spermatogenesis. This cell can be differentiation induced into various lines of cells, like ES cells and shows properties of, for example, generation of a chimeric mouse by transplantation into a mouse blastocyst and the like (Kanatsu-Shinohara M. et al. (2003) Biol. Reprod., 69: 612-616; Shinohara K. et al. (2004), Cell, 119: 1001-1012). It is self-renewable in a culture medium containing a glial cell line-derived neurotrophic factor (GDNF), and can produce a germ stem cell by repeating passages under culture conditions similar to those for ES cells (Masanori Takehashi et al., (2008), Experimental Medicine, Vol. 26, No. 5 (Suppl.), pp. 41 to 46, YODOSHA (Tokyo, Japan)).

EG cell is a cell having pluripotency similar to that of ES cells, which is established from a primordial germ cell at the prenatal period. It can be established by culturing a primordial germ cell in the presence of a substance such as LIF, bFGF, a stem cell factor, or the like (Matsui Y. et al. (1992), Cell, 70: 841-847; J.L. Resnick et al. (1992), Nature, 359: 550-551).

Muse cell is an in vivo endogeneous non-neoplastic pluripotent stem cell and can be produced, for example, by the method described in WO 2011/007900. In more detail, Muse cell is a cell having pluripotency, which is obtained by subjecting a fibroblast or a bone marrow stromal cell to a trypsin treatment for a long time, preferably 8 hr or 16 hr, and thereafter culturing the cells in a suspended state, and positive for SSEA-3 and CD105.

The aforementioned step (0) is not particularly limited as long as pluripotent stem cells can be induced to differentiate into cardiomyocytes. For example, differentiation into cardiomyocytes can be induced by culturing pluripotent stem cells in a medium for cardiomyocyte differentiation. In one embodiment of the present invention, the aforementioned step (0) may include (0-1) step of inducing differentiation of pluripotent stem cells into mesodermal cells, and (0-2) step of inducing differentiation of the mesodermal cells into cardiomyocytes. In the present specification, the "medium for cardiomyocyte differentiation" means a medium containing a factor that promotes induction of differentiation into cardiomyocytes such as cytokine and the like (hereinafter sometimes referred to as "cardiomyocyte differentiation-inducing factor") and a basal medium. The above-mentioned factor that promotes induction of differentiation into cardiomyocytes encompasses a factor necessary for inducing differentiation into intermediate cells (e.g., mesodermal cell and the like) in the differentiation induction process from pluripotent stem cells into cardiomyocytes.

Examples of the basal medium to be used in the present invention include StemFit (e.g., StemFit AK03N, StemFit AK02N) (Ajinomoto Co., Inc.), StemPro-34 (Thermo Fisher Scientific), PECM (Primate ES Cell medium), GMEM (Glasgow Minimum Essential medium), IMDM (Iscove's Modified Dulbecco's medium), 199 medium, Eagle's Minimum Essential medium (EMEM), αMEM, Dulbecco's modified Eagle's medium (DMEM), Ham's F12 medium, RPMI 1640 medium, Fischer's medium, and a combined medium thereof, and the like.

It is possible to appropriately add ROCK inhibitors (e.g., Y-27632, Fasudil/HA1077, SR3677, GSK269962, H-1152, Wf-536, etc.), serums (e.g., bovine embryo serum (FBS), human serum, horse serum, etc.) or serum substitutes, insulins, various vitamins (e.g., vitamin C (e.g., ascorbic acid)), various amino acids such as L-glutamine, non-essential amino acid, and the like, 2-mercaptoethanol, thioglycerol (e.g., α-monothioglycerol (MTG)), various cytokines, stem cell factors (SCF (Stem cell factor)), activin, etc.), various hormones, various growth factors (leukemia inhibitory factor (LIF), basic fibroblast growth factor (bFGF), TGF-β, etc.), various extracellular matrixs, various cell adhesion molecules, antibiotics such as penicillin/streptomycin, puromycin, and the like, pH indicators such as phenol red and the like, and the like to the basal medium. Examples of the serum substitute include albumin, transferrin, fatty acid, insulin, collagen precursor, trace element, Knockout Serum Replacement (KSR), ITS-supplement, and mixtures thereof, and the like.

In the present invention, the "Vitamin Cs" means L-ascorbic acid and derivatives thereof, and "L-ascorbic acid derivative" means derivatives that become vitamin C by enzymatic reaction in the living body. Examples of the derivatives of L-ascorbic acid to be used in the present invention include vitamin C phosphate (e.g., ascorbic acid 2-phosphate), ascorbic acid glucoside, ascorbyl ethyl, vitamin C ester, ascorbyl tetrahexyldecanoate, ascorbyl stearate, and ascorbyl 2-phosphate 6-palmitate. Preferred is vitamin C phosphate (e.g., ascorbic acid 2-phosphate). Examples of the vitamin C phosphate include salts of L-ascorbic acid phosphate such as L-ascorbic acid phosphate Na and L-ascorbic acid phosphate Mg.

The induced pluripotent stem cells or embryoid bodies may be cultured by adherent culture or suspension culture. In cases of adherent culture, the culturing may be carried out in a culture vessel coated with an extracellular matrix component, and/or may be co-cultured with feeder cells. While the feeder cell is not particularly limited, for example, fibroblast (mouse embryo fibroblast (MEF), mouse fibroblast (STO), and the like) can be mentioned. Feeder cells are preferably inactivated by a method known per se, for example, radiation (gamma-ray and the like) irradiation, treatment with anticancer agent (mitomycin C and the like), or the like. As the extracellular matrix component, fibrous proteins such as Matrigel (Niwa A, et al., PLoS One.6(7): e22261, 2011), gelatin, collagen, elastin, and the like, glucosaminoglycan and proteoglycan such as hyaluronic acid, chondroitin sulfate, and the like, cell adhesion proteins such as fibronectin, vitronectin, laminin, and the like, and the like can be mentioned.

The culture temperature conditions are not particularly limited. The temperature is, for example, about 37° C to 42° C, preferably about 37° C to 39° C. The culture may be carried out under low-oxygen conditions, and the low-oxygen condition in the present invention means, for example, oxygen concentration of 15%, 10%, 9%, 8%, 7%, 6%, 5% or lower than these.

Suspension culture means culturing cells in a state of being non-adhesive to the culture container, and is not particularly limited. It can be performed using a culture container free of an artificial treatment (e.g., coating treatment with extracellular matrix or the like) in order to improve adhesiveness to cells, or a culture container artificially treated to suppress adhesion (e.g., coating treatment with poly-hydroxyethyl methacrylic acid (poly-HEMA) or non-ionic surface-active activity polyol (Pluronic F-127, etc.)). For example, suspension culture may be performed using an incubator provided with an impeller, such as single-use bioreactor (Biott Corporation), single-use bioreactor (Thermo Fisher), single-use bioreactor (Sartorius Stedim), single-use bioreactor (GE Healthcare Life Sciences), or the like. The kind and the stirring rate of the incubator to be used can be appropriately selected by those of ordinary skill in the art according to the type of cells to be cultured. For example, the stirring rate may be, though not limited to, 0 to 100 rpm, 20 to 80 rpm, or 45 to 65 rpm.

During the suspension culture, it is preferable to form an embryoid body (EB) and culture the same. Therefore, the aforementioned step (0) may include a step of forming an embryoid body from pluripotent stem cells. In such step, it is preferable to dissociate colony-forming pluripotent stem cells into single cells and then form the embryoid body. In the step of dissociating pluripotent stem cells, the cells forming a population by adhering to each other are dissociated (separated) into individual cells. The method for dissociating pluripotent stem cells includes, for example, a method of mechanical dissociation, and a dissociation method using a dissociation solution having protease activity and collagenase activity (e.g., Accutase (trade mark), Accumax (trade mark), and the like) or a dissociation solution having collagenase activity alone. Preferably, a method of dissociating pluripotent stem cells by using a dissociation solution having protease activity and collagenase activity (particularly preferably Accumax (trade mark)) is used. The medium used in the above-mentioned step preferably contains thioglycerol, L-glutamine and/or ascorbic acid.

Cardiomyocyte differentiation-inducing factors used in the above-mentioned step (0-1) include, for example, Wnt signal activating substance, activin A, BMP4, and bFGF, and these may be used alone or in combination. In one embodiment of the present invention, activin A, BMP4, and bFGF are used in combination. In addition, the medium used in the above-mentioned step (0-1) preferably contains thioglycerol, L-glutamine and/or ascorbic acid.

In the present specification, the "Wnt signal activator" means a substance that activates the Wnt signal pathway. Examples of the Wnt signal activator include Wnt protein, GSK3β inhibitor (e.g., BIO, CHIR99021, etc.), and the like. These may be used alone or in combination. When a Wnt signal activator is used, the concentration thereof in the medium is not particularly limited. When BIO or CHIR99021 is used as the Wnt signal activator, it is preferably used at a final concentration of 100 nM to 100 µM, preferably 1 µM to 10 µM, in the medium.

When activin A is used, the concentration thereof in the medium is preferably 1 ng/ml to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

When BMP4 is used, the concentration thereof in the medium is preferably 1 ng/ml to 1 µg/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, 100 ng/ml, 200 ng/ml, 300 ng/ml, 400 ng/ml, 500 ng/ml, 600 ng/ml, 700 ng/ml, 800 ng/ml, 900 ng/ml, or 1 µg/ml.

When bFGF is used, the concentration thereof in the medium is preferably 1 ng/ml to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

The period of the above-mentioned step (0-1) is not particularly limited as long as mesodermal cells are obtained. It is preferably not less than 12 hr (e.g., 1 day, 2 days, or longer), or may be not more than 6 days (e.g., 5 days, 4 days, 3 days, or shorter). In addition, whether or not mesodermal cells are obtained may be monitored, and in this case, it can be determined by the expression of the mesoderm marker gene. Examples of the mesoderm marker gene include T, MIXL1, NODAL, and the like.

Examples of the cardiomyocyte differentiation-inducing factor used in the above-mentioned step (0-2) include Wnt inhibitor and VEGF. These may be used alone or in combination. The medium used in the above-mentioned step (0-2) preferably contains thioglycerol, L-glutamine and/or ascorbic acid.

In the present specification, the "Wnt inhibitor" means a substance that inhibits signal transduction induced by the binding of Wnt to receptors subjected to the accumulation of β-catenin. It may be a substance that inhibits the binding to the Frizzled family of receptors or a substance that promotes the degradation of β-catenin. Examples of the Wnt inhibitor include DKK1 protein (e.g., in the case of human, NCBI accession number: NM_012242), sclerostin (e.g., in the case of human, NCBI accession number: NM_025237), IWR-1 (Merck Millipore), IWP-2 (Sigma-Aldrich), IWP-3 (Sigma-Aldrich), IWP-4 (Sigma-Aldrich), PNU-74654 (Sigma-Aldrich), XAV939 (Sigma-Aldrich), derivatives thereof, and the like. Among these, IWP-3 or IWP-4 is preferred. Only one kind of Wnt inhibitor may be used or plural kinds thereof may be used in combination.

When a Wnt inhibitor is used, its concentration in the medium is preferably 1 nM to 50 µM, and is, for example, 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though not limited to these. It is more preferably 1 µM.

When VEGF is used, its concentration in the medium is preferably 1 to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

In the above-mentioned step (0-2), a BMP inhibitor and/or a TGFβ inhibitor may be further added as a cardiomyocyte differentiation-inducing factor to the basal medium. In the present specification, xamples of the "BMP inhibitor" include proteinaceous inhibitors such as Chordin, Noggin, Follistatin, and the like, Dorsomorphin (6-[4-(2-piperidin-1-yl-ethoxy)phenyl]-3-pyridin-4-yl-pyrazolo[1,5-a]pyrimidine) and a derivative thereof (P. B. Yu et al. (2007), Circulation, 116: II#60; P. B. Yu et al. (2008), Nat. Chem. Biol., 4: 33-41; J. Hao et al. (2008), PLoS ONE, 3(8): e2904, LDN-193189 (4-(6-(4-(piperazin-1-yl)phenyl)pyrazolo[1,5-a]pyrimidin-3-yl)quinoline), and the like. Among these, Dorsomorphin is preferred. Only one kind of BMP inhibitor or TGFβ inhibitor may be used or plural kinds thereof may be used in combination.

When a BMP inhibitor is used, its concentration in the medium is preferably 1 nM to 50 µM, and is, for example, 1 nM, 10 nM, 50 nM, 100 nM, 500 nM, 600 nM, 700 nM, 800 nM, 900 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though not limited to these.

In the present specification, the TGFβ inhibitor means a substance that inhibits signal transduction from the binding of TGFβ to a receptor, leading to SMAD. It may be a substance that inhibits the binding to ALK family of receptors, or a substance that inhibits phosphorylation of SMAD by ALK family. Examples of the TGFβ inhibitor include Lefty-1 (e.g., mouse: NM_010094, human: NM_020997 in NCBI Accession No.), SB431542, SB202190 (all R.K. Lindemann et al., Mol. Cancer, 2003, 2:20), SB505124 (GlaxoSmithKline), NPC30345, SD093, SD908, SD208 (Scios), LY2109761, LY364947, LY580276 (Lilly Research Laboratories), A-83-01 (WO 2009146408), derivatives thereof, and the like. Among them, SB431542 is preferred.

When a TGFβ inhibitor is used, its concentration in the medium is preferably 1 nM to 50 µM, and is, for example, 1 nM, 10 nM, 50 nM, 100 nM, 500 nM, 750 nM, 1 µM, 2 µM, 3 µM, 4 µM, 5 µM, 5.2 µM, 5.4 µM, 5.6 µM, 5.8 µM, 6 µM, 7 µM, 8 µM, 9 µM, 10 µM, 15 µM, 20 µM, 25 µM, 30 µM, 40 µM, or 50 µM, though not limited to these.

The period of the above-mentioned step (0-2) is not particularly limited as long as cardiomyocytes are obtained. It is, for example, not less than 1 day (e.g., 1 day, 2 days, 3 days, 4 days, 5 days, 6 days, 7 days, or longer). Since long-term culture does not affect the establishment of cardiomyocytes, the upper limit is not particular set, but it is typically not more than 40 days. In addition, whether or not cardiomyocytes are obtained may be monitored. In this case, it can be confirmed by the number of beating cardiomyocytes, expression of the markers of cardiomyocytes, expression of ion channels, response to electric physiological stimulation, or the like.

The aforementioned step (0) may further contain step (0-3) of culturing cardiomyocytes, which are obtained in step (0-2), in the presence or absence of VEGF and/or bFGF. The medium used in this step preferably contains thioglycerol, L-glutamine and/or ascorbic acid. In addition, the medium used in this step may also contain a compound for myocardial maturation (e.g., N-(1,1-dioxo-2,3-dihydro-1H-1-benzothiophen-5-yl)-2-(4-{5-[1-oxo-5-(piperidin-1-yl)-1,3-dihydro-2H-isoindol-2-yl]-1H-benzoimidazol-2-yl}phenoxy)acetamide) and/or a multikinase inhibitor (e.g., 2-(4-{3-[3-(2-amino-2-phenylethoxy)-4-cyanophenyl]pyrazolo[1,5-a]pyrimidin-6-yl}-1H-pyrazol-1-yl)-N-(2-methoxyethyl) acetamide).

When VEGF is used in step (0-3), its concentration in the medium is preferably 1 to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml.

When bFGF is used in step (0-3), its concentration in the medium is preferably 1 to 100 ng/ml, and is, for example, 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 30 ng/ml, 40 ng/ml, 50 ng/ml, 60 ng/ml, 70 ng/ml, 80 ng/ml, 90 ng/ml, or 100 ng/ml. It is more preferably 5 ng/ml.

The period of the above-mentioned step (0-3) is not particularly limited. For example, it is not less than one day (e.g., 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, or longer). Since long-term culture does not affect the establishment of cardiomyocytes, the upper limit is not particular set, but it is typically not more than 60 days. The above-mentioned step (0-3) may improve efficiency of differentiation into cardiomyocytes.

In addition, embryoid bodies may be dissociated by a method similar to that described above, prior to the above-mentioned step (0-2) or step (0-3).

The method specifically described above is merely an example, and the method is not limited to the above-mentioned method. For example, a method of coculturing END2 cells, which are mouse-derived supporting cells, and pluripotent stem cells (Mummery, C., et al., Circulation. 107(21), 2733-40 (2003)), a method of inducing cardiomyocytes by culturing embryoid bodies with BMP4, FGF2, insulin, and serum (Paul, W B., et al., PLoSone. 6(4), e18293 (2011)), and the like can be mentioned. In addition, a method of inducing differentiation of cardiomyocytes without using cytokine in adhesion culture (Lian X, et al., Proc Natl Acad Sci USA., 2012 July 3; 109(27): E1848-57), a method of inducing differentiation of cardiomyocytes by adhesion culture and suspension culture in combination without using cytokine (Minami I, et al., Cell Rep., 2012 Nov 29; 2(5): 1448-60), and the like may also be used.

A cell population with high purity of cardiomyocytes, compared with the cell population before contact with an HDAC inhibitor, can be produced by bringing a cell population containing cardiomyocytes obtained as mentioned above into contact with the HDAC inhibitor, and then culturing the cell population. That is, cardiomyocytes can be purified using an HDAC inhibitor. Therefore, in another embodiment of the present invention, a method for purifying cardiomyocytes, including (1') a step of bringing an HDAC inhibitor into contact with a cell population containing cardiomyocytes and cells other than cardiomyocytes, the cell population being obtained by culturing pluripotent stem cells in a medium for cardiomyocyte differentiation, and (2') a step of culturing the cell population (hereinafter also to be referred to as "the purification method of the present invention") is provided. In a still another embodiment, a method for reducing cells other than cardiomyocytes from a cell population containing cardiomyocytes and cells other than the cardiomyocytes, the method including the above-mentioned steps (1') and (2') (hereinafter also to be referred to as "the method for reducing non-cardiomyocytes of the present invention") is also provided.

In the present specification, purifying cardiomyocytes means that the proportion of cardiomyocytes in a cell population (number of cardiomyocytes in cell population/total number of cells in cell population) increases because the rate of decrease in the cell count of cells other than cardiomyocytes ("cell count" means viable cell count, hereinafter the same) exceeds the rate of decrease in cardiomyocytes, or the proliferation of cells other than cardiomyocytes is inhibited and thus the proliferation rate of cardiomyocytes exceeds the proliferation rate of cells other than cardiomyocytes, each due to an HDAC inhibitor. Therefore, an increase in the proportion of cardiomyocytes during the purification of cardiomyocytes is distinguished from an increase in the proportion of cardiomyocytes due to the promotion of differentiation induction from cardiac progenitor cells into cardiomyocytes or inhibition of differentiation induction from cardiac progenitor cells into cells other than cardiomyocytes.

As shown in the Examples described below, it was shown that an HDAC inhibitor treatment causes a higher rate of decrease in the number of pluripotent stem cells than the rate of decrease in the number of cardiomyocytes, and that an HDAC inhibitor treatment increases the proportion of cardiomyocytes in the cell population. Without wishing to be bound by any theory, it is assumed that the decrease in the cell count due to an HDAC inhibitor is the result of the induction of cell apoptosis by the HDAC inhibitor. In addition, it is assumed that the proportion of undifferentiated cells, such as pluripotent stem cell and the like, contained in the cell population decreases by using an HDAC inhibitor, or the proportion of cardiomyocytes in the cell population increased by removing undifferentiated cells from the cell population by using an HDAC inhibitor. Therefore, in another embodiment of the present invention, a method for removing or reducing undifferentiated cells from a cell population containing cardiomyocytes and contaminated with the undifferentiated cells, including (I) a step of contacting a histone deacetylase inhibitor with the cell population, and (II) a step of culturing the cell population (hereinafter also to be referred to as "the undifferentiated cell removal method of the present invention") is provided.

In the present specification, the "undifferentiated cell" means a cell that retains differentiation potency, that is, pluripotency, multipotency, oligopotency or unipotency. Specifically, the undifferentiated cell includes the aforementioned pluripotent stem cell, mesodermal cell having differentiation potency, cardiac progenitor cell, and the like.

In the aforementioned steps (1), (1'), and (I), the period of contact of a cell population containing cardiomyocytes and an HDAC inhibitor is not particularly limited. For example, it is preferably not less than 1 hr (e.g., 2 hr, 3 hr, 5 hr, 12 hr, 1 day, 2 days, 3 days, or longer). Since long-term culture does not affect the establishment of cardiomyocytes or cardiac progenitor cells, the upper limit is not particular set, but it is typically not more than 60 days (e.g., 50 days, 40 days, 30 days, 20 days, 14 days, 13 days, 12 days, 11 days, or shorter). The contact between a cell population containing cardiomyocytes and an HDAC inhibitor may be performed by adding an HDAC inhibitor to a medium containing the cell population, or by seeding the cell population in a medium previously supplemented with an HDAC inhibitor. The timing of contacting the HDAC inhibitor is not particularly limited as long as the cell population contains cardiomyocytes. For example, contacting in the above-mentioned step (0-2) or (0-3) is preferred. Based on the start date of induction of differentiation of pluripotent stem cells, contacting on or after 7 days from the start of differentiation induction is preferred.

HDAC to be inhibited by the HDAC inhibitor used in the present invention may be any of class I HDAC (e.g., HDAC1, HDAC2, HDAC3, HDAC8), class II HDAC (e.g., HDAC4, HDAC5, HDAC6, HDAC7, HDAC9, HDAC10), class III HDAC (e.g., SirT1, SirT2, SirT3, SirT4, SirT5, SirT6, SirT7), and class IV HDAC (e.g., HDAC11). It is preferably class I or II HDAC, more preferably class I HDAC, particularly an inhibitor to HDAC1 or HDAC2. In the present specification, the inhibitor to class I HDAC may have other activities, for example, inhibitory activity to HDAC of a class other than class I, and the like as long as it has inhibitory activity to class I HDAC, and may have specific inhibitory activity to class I HDAC. The same applies to an inhibitor to HDAC of other class.

Examples of the inhibitor to HDAC1 to be used in the present invention include Trichostatin A, CI994 (Tacedinaline), Quisinostat (JNJ-26481585), CUDC-907, PCI-24781 (Abexinostat), RG2833 (RGFP109), Romidepsin (FK228, Depsipeptide), Resminostat, Pracinostat (SB939), Rocilinostat (ACY-1215), Mocetinostat (MGCD0103), CAY10603, Entinostat (MS-275), 4SC-202, HPOB, PCI-34051, Tubastatin A HCl, and the like.

Examples of the inhibitor to HDAC2 include Trichostatin A, Quisinostat (JNJ-26481585), CUDC-907, CUDC-101, PCI-24781 (Abexinostat), Romidepsin (FK228, Depsipeptide), Rocilinostat (ACY-1215), Pracinostat (SB939), Mocetinostat (MGCD0103), 4SC-202, HPOB, and the like.

Examples of the inhibitor to HDAC3 include Trichostatin A, RGFP966, CUDC-907, Quisinostat (JNJ-26481585), RG2833 (RGFP109), PCI-24781 (Abexinostat), CUDC-101, Pracinostat (SB939), Resminostat, Rocilinostat (ACY-1215), 4SC-202, Mocetinostat (MGCD0103), HPOB, Entinostat (MS-275), Droxinostat, and the like.

Examples of the inhibitor to HDAC4 include Trichostatin A, Tasquinimod, Quisinostat (JNJ-26481585), LMK-235, CUDC-101, Pracinostat (SB939), TMP269, CUDC-907, Rocilinostat (ACY-1215), and the like.

Examples of the inhibitor to HDAC5 include Quisinostat (JNJ-26481585), LMK-235, CUDC-101, Pracinostat (SB939), TMP269, CUDC-907, Rocilinostat (ACY-1215), and the like.

Examples of the inhibitor to HDAC6 include Trichostatin A, CAY10603, Tubacin, Rocilinostat (ACY-1215), Nexturastat A, Tubastatin A HCl, Tubastatin A, HPOB, CUDC-101, PCI-24781 (Abexinostat), CUDC-907, Resminostat, Quisinostat (JNJ-26481585), Pracinostat (SB939), Droxinostat, PCI-34051, and the like.

Examples of the inhibitor to HDAC7 include TMP269, Quisinostat (JNJ-26481585), Pracinostat (SB939), CUDC-101, CUDC-907, Rocilinostat (ACY-1215), and the like.

Examples of the inhibitor to HDAC8 include PCI-34051, Quisinostat (JNJ-26481585), CUDC-101, Rocilinostat (ACY-1215), Pracinostat (SB939), CUDC-907, PCI-24781 (Abexinostat), Tubastatin A HCl, Droxinostat, HPOB, and the like.

Examples of the inhibitor to HDAC9 include TMP269, Quisinostat (JNJ-26481585), CUDC-101, Pracinostat (SB939), CUDC-907, and the like.

Examples of the inhibitor to HDAC10 include Trichostatin A, Quisinostat (JNJ-26481585), CUDC-907, PCI-24781 (Abexinostat), CUDC-101, Pracinostat (SB939), HPOB, PCI-34051, and the like.

Examples of the inhibitor to HDAC11 include Quisinostat (JNJ-26481585), CUDC-907, Pracinostat (SB939), Mocetinostat (MGCD0103), and the like.

As the inhibitor to class III HDAC, Sirtinol, SirReal2, Nicotinamide (Vitamin B3), Selisistat (EX 527), Thiomyristoyl, Salermide, OSS_128167, AK 7, 3-TYP, Tenovin-1, Rocilinostat (ACY-1215), Inauhzin, and the like can be mentioned.

In addition, a nonselective HDAC inhibitor may also be used and, as the inhibitor, Vorinostat (SAHA), Panobinostat (LBH589), Belinostat (PXD101), and the like can be mentioned. Among them, FK228, Entinostat, Trichostatin A, or Panobinostat is preferred. Only one kind of HDAC inhibitor may be used or plural kinds thereof may be used in combination.

The concentration of the HDAC inhibitor in the medium can be appropriately selected by those of ordinary skill in the art. For example, it is preferably 0.1 nM to 10 µM, and is specifically 0.5 nM, 1 nM, 2 nM, 3 nM, 5 nM, 10 nM, 20 nM, 30 nM, 40 nM, 50 nM, 0.1 µM, 0.2 µM, 0.3 µM, 0.4 µM, 0.5 µM, 1.0 µM, 1.5 µM, 2 µM, 3 µM, 4 µM, 5 µM, 6 µM, 7 µM, 8 µM, 9 µM, and 10 µM. The concentration can also be changed as appropriate according to the kind of the compound. For example, when Trichostatin A is used, 0.1 µM to 10 µM is preferred; when Panobinostat is used, 0.1 to 3 µM is preferred; when FK228 is used, 1 nM to 1 µM is preferred; and when Entinostat is used, 0.1 µM to 10 µM is preferred, though not limited to these concentrations.

The method for culturing the cell population in the aforementioned steps (2), (2'), and (II) is the same as that in the above-mentioned (0-3). The culture period is the same, and the culture may be continued at least while the cell population is in contact with the HDAC inhibitor.

### 2. Cell population containing cardiomyocytes

The present invention also provides a cell population containing cardiomyocytes (hereinafter also to be referred to as "the cell population of the present invention") which is obtained by the production method, the purification method, the method for reducing non-cardiomyocytes, or the undifferentiated cell removal method of the present invention. As described above, the cell population of the present invention contains cardiomyocytes with high purity. High purity means that the ratio of cardiomyocytes in the cell population (number of cardiomyocytes in the cell population/total number of cells in the cell population) is specifically not less than 80% (e.g., 85%, 90%, 91%, 92%, 93%, 94%, 95%, 96%, 97%, 98%, 99%, or higher). When the above-mentioned cell population and other cells or cell populations such as mesenchymal stem cells and the like are mixed and used, the ratio of the above-mentioned cardiomyocytes is naturally the one before mixing with other cells or cell populations. In a preferred embodiment, the cell population of the present invention contains cardiomyocytes at a higher percentage than a cell population obtained by a conventional method of inducing cardiomyocytes from pluripotent stem cells. Such cell populations may be further purified by cell sorting or the like, and such purified cell population is also encompassed in the "the cell population of the present invention".

### 3. Agent for cell transplantation therapy

The present invention also provides an agent for cell transplantation therapy containing the cell population of the present invention (hereinafter also to be referred to as "the cell transplantation therapy agent of the present invention"). The cell transplantation therapy agent of the present invention may be used for autologous transplantation or allotransplantation. In addition, it may be used in combination with other medicaments such as immunosuppressant. As mentioned above, since the cell population of the present invention contains cardiomyocytes with high purity, it is suitably used as a starting material of an agent for cell transplantation therapy, and the cell population of the present invention and the cell transplantation therapy agent of the present invention are useful for the prophylaxis or treatment of cardiac diseases. Therefore, a method for treating or preventing cardiac diseases, including administering or transplanting an effective amount of the cell population or the cell transplantation therapy agent of the present invention to a mammal (e.g., human, mouse, rat, monkey, bovine, horse, swine, dog, etc.) as the target of prophylaxis or treatment of is also encompassed in the present invention. Examples of the cardiac disease to be the target of prophylaxis or treatment of include, but are not limited to, diseases such as cardiac failure, ischemic cardiac diseases, myocardial infarction, cardiomyopathy, myocarditis, hypertrophic cardiomyopathy, dilated phase of hypertrophic cardiomyopathy, dilated cardiomyopathy, and the like, deficiency due to disorder, and the like.

From the aspect that rejections do not occur when the cell population of the present invention is used as an agent for cell transplantation therapy, it is desirable to use a cell population containing cells derived from iPS cells established from somatic cells that have the same or substantially the same HLA genotype as the individual who receives transplantation. As used herein, "substantially the same" means that the HLA genotypes match to the extent that an immunosuppressant can suppress an immune response to the transplanted cells. For example, it refers to a somatic cell with an HLA type in which 3 loci of HLA-A, HLA-B, and HLA-DR or 4 loci including HLA-C are matched. In addition, it is also possible to embed the cells in a capsule such as polyethylene glycol or silicon, or a porous container, etc. and transplant the cells while avoiding rejections.

The cell population of the present invention can be produced as a parenteral formulation such as injection, suspension, drip transfusion, or the like, in a mixture with a pharmaceutically acceptable carrier, by a conventional means. Examples of the pharmaceutically acceptable carrier that can be contained in the parenteral preparation include aqueous liquids for injection, such as isotonic solution containing physiological saline, glucose, and other auxiliary drugs (e.g., D-sorbitol, D-mannitol, sodium chloride, and the like). The agent for cell transplantation therapy of the present invention may be formulated with, for example, a buffering agent (e.g., phosphate buffer solution, sodium acetate buffer solution), a soothing agent (e.g., benzalkonium chloride, procaine hydrochloride, and the like), a stabilizer (e.g., human serum albumin, polyethylene glycol, and the like), a preservative, an anti-oxidizing agent, or the like. When the agent for transplantation therapy of the present invention is formulated as an aqueous suspension, a cell population containing cardiomyocytes may be suspended in the above-mentioned aqueous solution at about 1×10⁶ to about 1×10⁸ cells/mL. It can also be administered with a scaffolding material that promotes engraftment. Examples of the scaffolding material include, but are not limited to, components of biological origin such as collagen and the like, and synthetic polymers such as polylactic acid and the like that substitute for the components.

Alternatively, cardiac diseases may be treated by adhering a sheet formed from the obtained cardiomyocytes to the heart of patients. When a cardiac muscle sheet is administered, the sheet is disposed to cover the desired area. Here, disposing to cover the desired area can be performed using a technique well known in the pertinent field. During the disposing, when the desired area is large, the sheet may also be placed to surround the tissue. In order to achieve the desired effect, the administration may include several times of disposing on the same area. When disposing is performed several times, it is desirable to secure sufficient time so that the desired cells will be engrafted in the tissue to allow for angiogenesis. The mechanism of such treatment of cardiac diseases may be an effect resulting from the engraftment of a cardiac muscle sheet, or an indirect action not based on cell engraftment (e.g., effect by recruiting recipient-derived cells to damaged site caused by secretion of an attractant). When a cardiac muscle sheet is used in the treatment of cardiac diseases, it may contain a cell scaffolding material (scaffold) such as collagen, fibronectin, laminin, or the like, in addition to cardiomyocytes. Alternatively, it may contain any cell type (possibly in plurality) in addition to cardiomyocytes. The number of cardiomyocytes used for the treatment of cardiac diseases is not particularly limited as long as the cardiac muscle sheet to be administered exhibits an effect in the treatment of cardiac diseases and can be appropriately increased or decreased for adjustment according to the size of the affected part and the size of the body.

In still another embodiment, the cell population of the present invention can also be used for drug screening or drug cardiotoxicity evaluation for the treatment of cardiac disease. For example, by administering a test drug to the cell population of the present invention, and then measuring the response of the cardiomyocytes, the effect and toxicity of the test drug can be evaluated.

While the present invention is further explained specifically in the following Examples, the scope of the present invention is not limited by such Examples.

### [Example]

### Experimental Example 1. Screening for compound with purification action on cardiomyocytes

Based on differences in the proliferation between cardiomyocytes and non-cardiomyocytes, it was assumed that the sensitivity to compounds that target cell proliferation and cell cycle varies. Thus, screening for a compound with a purification action on cardiomyocytes was performed using an iPS cell line for research.

### <iPS cell line>

To detect cardiomyocyte maturation, a double knock-in human iPS cell line (reporter iPS cell line) was produced by inserting the reporter protein sequence of EmGFP (SEQ ID NO: 1) into the TNNI1 gene locus and the reporter protein sequence of mCherry (SEQ ID NO: 2) into the TNNI3 gene locus. Maintenance culture of the above-mentioned reporter iPS cell line was performed according to the conventional method (Okita K, et al. Stem Cells. 2012 Nov 29. doi: 10.1002/stem.1293). The above-mentioned human iPS cell line was established by PBMC (LP_167, Sample ID: 20130318) purchased from CTL and an episomal vector (loaded gene; OCT3/4, KLF4, SOX2, L-MYC, LIN28, mouse p53DD) (reference document; Okita K, et al. Stem Cells. 2012 Nov 29. doi: 10.1002/stem.1293).

### <Induction of differentiation into cardiomyocytes>

Induction of differentiation into cardiomyocytes was performed according to the method described in a paper (Miki et al, Cell Stem Cell. 2015 Jun 4; 16. doi: 10.1016/j.stem. 2015.04.005.) and using a 6-well plate. For brief explanation, induction of differentiation into cardiomyocytes was performed by treating the reporter iPS cell line for 4 to 5 min with TrypLE select (Life Technologies) diluted to 1/2 with 0.5 mM EDTA/PBS, detaching the cells with a cell scraper (IWAKI), and dissociating the cells into single cells by pipetting. The medium was removed by centrifugation at 1,000 rpm for 5 min, and the obtained cells were seeded at 2×10⁶ cells per well of a 6-well plate and cultured in StemPro34 medium, supplemented with 1% L-glutamine, transferrin 150 µg/mL, ascorbic acid 50 µg/mL (sigma), monothioglycerol 4×10⁻⁴ M, 10 µM Y-27632, 2 ng/ml BMP4 (R&D), and 0.5% Growth Factor Reduced Matrigel, at 1.5 mL/well under 37° C, 5% oxygen conditions to form embryoid bodies (day 0).

The next day (day 1), 1.5 mL of StemPro34 medium supplemented with 1% L-glutamine, transferrin 150 µg/mL, ascorbic acid 50 µg/mL (sigma), monothioglycerol 4×10⁻⁴ M, 2 ng/ml BMP4 (R&D) activin A 12 ng/ml, bFGF 5 ng/ml, and BMP4 18 ng/ml was added to each well, and the cells were cultured under 37° C, 5% oxygen conditions for two more days.

Successively (day 3), the 6-well plate was stood while tilting to allow embryoid bodies to form sediments, 80 to 90% of the medium was removed, and IMDM (1.5 mL) was added to each well. The well plate was stood while tilting again to allow embryoid bodies to form sediments, 80 to 90% of the medium was removed, and the cells were cultured in StemPro34 medium supplemented with 1% L-glutamine, transferrin 150 µg/mL, ascorbic acid 50 µg/mL (sigma), monothioglycerol 4×10⁻⁴ M, 10 ng/ml VEGF, 1 µM IWP-3, 0.6 µM Dorsomorphin, and 5.4 µM SB431542, under 37° C, 5% oxygen conditions for 3 days.

Successively (day 6), the 6-well plate was stood while tilting to allow embryoid bodies to form sediments, 80 to 90% of the medium was removed, and StemPro34 medium supplemented with 1% L-glutamine, transferrin 150 µg/mL, ascorbic acid 50 µg/mL (sigma), monothioglycerol 4×10⁻⁴ M, and 5 ng/ml VEGF was added. The cells were cultured for 10 days under 37° C, 5% oxygen conditions. During this period, the medium was exchanged with a medium under the same conditions once every 2 to 3 days. The cells were cultured under 37° C, 210 oxygen conditions on day 10 and thereafter.

### <Dissociation into single cells of embryoid body and compound screening>

On day 22 from the start of differentiation, a 6-well plate containing embryoid body was tilted, stood for 1 to 2 min until a sediment of the embryoid body was formed at the edge of the well, and the supernatant was aspirated while avoiding sucking the embryoid body. Thereafter, 2 mL of PBS was added. The plate was tilted in the same manner as described above, stood for 1 to 2 min, and PBS was aspirated while avoiding sucking the embryoid body. Using Papain Dissociation System (Worthington), EB was dissociated into single cells, subjected to centrifugation (70 g, 6 min), and suspended in 2% FBS/PBS, and GFP-positive cells were separated by a flow cytometer. After centrifugation, the supernatant was removed, and the cells were resuspended in a medium for myocardial differentiation based on StemPro34 medium (Stempro 34 mixed with ascorbic acid 50 µg/mL, L-glutamine 2 mM, transferrin 150 mg/mL, monothioglycerol 4×10⁻⁴ M, VEGF 5 ng/ml) (100 mL). The resuspended cells were seeded in a CellCarrier-384 plate Ultra Microplate previously coated with fibronectin at 2.0×10³ cells/well. The cell population obtained by the culture is hereinafter referred to as cardiomyocytes.

In addition, the iPS cell line was treated for 4 to 5 min with 0.5×TrypLE select (Life Technologies, diluted to 1/2 with 0.5 mM EDTA/PBS), the cells were detached with a cell scraper (IWAKI), and dissociated into single cells by pipetting. The medium was removed by centrifugation (1,000 rpm, 5 min), and the cells were resuspended in StemFit AK02 medium supplemented with Y-27632 10 µM, and seeded at 2.0×10³ cells/well on CellCarrier-384 Ultra Microplate (PerkinElmer/6057300) coated with iMatrix-511 (Nippi).

Two days after seeding, an evaluation compounds were added to cardiomyocytes and single-celled iPS cells, and the cells were cultured for two days. After 48 hr from the treatment with compound, viable cell count was evaluated by measuring the intracellular ATP level by using CellTiter-Glo (PerkinElmer). The results of the compounds (HDAC inhibitors) that markedly decreased the number of viable iPS cells (ATP level) compared to the number of viable cardiomyocytes (ATP level) as a result of screening are shown in Fig. 1. Fig. 1 shows relative values of iPS cells and cardiomyocytes when the ATP level of the DMSO control is 1000 (n=2).

### Experimental Example 2. Verification of purification action in clinical iPS cell line

Then, it was confirmed by ATP assay whether or not the HDAC inhibitors found in the screening of Experimental Example 1 also have a purification action on cardiomyocytes derived from clinical iPS cell line.

### <iPS cell line>

A clinical iPS cell line produced by the CiRA was used. Maintenance culture of iPS cell line was performed according to the conventional method (Okita K, et al. Stem Cells. 2012 Nov 29. doi: 10.1002/stem.1293).

### <Induction of differentiation into cardiomyocytes>

Induction of differentiation into cardiomyocytes was performed according to the method described in <Induction of differentiation into cardiomyocytes> of the above-mentioned Experimental Example 1.

### <Dissociation into single cells of embryoid body and compound screening>

On day 20, a 10 cm dish containing embryoid body was tilted, stood until a sediment of the embryoid body was formed, and the supernatant was aspirated while avoiding sucking the embryoid body. A solution of IMDM added with DNase 10 ug/mL and Liberase 100 µg/mL was added at 3 mL per one dish, and the mixture was stood at 37° C under general oxygen conditions for 1 hr. After 1 hr, a tube was stood for 1 to 2 min until a sediment of the embryoid body was formed, and the supernatant was aspirated while avoiding sucking the embryoid body. A solution of TrypLE select added with DNase 10 µg/mL was added at 2 mL per one dish, and the mixture was stood at 37° C under general oxygen conditions for 15 min. Thereafter, IMDM medium added with DNase 10 µg/mL was added at 2 mL per one dish. Single cells were obtained by pipetting and subjected to centrifugation (100 g, 4° C, 5 min). After centrifugation, the supernatant was removed, and the cells were suspended in I3 medium (same medium for myocardial differentiation in Experimental Example 1) and seeded in a fibronectin-coated 96-well plate at a seeding density of 2×10⁴ cells/well. The cell population obtained by the culture is hereinafter referred to as cardiomyocytes. A part of the cells was fixed, stained with an anti-sarcomeric α-actinin antibody, and the expression of sarcomeric α-actinin was analyzed with a flow cytometer. As a result, the sarcomeric α-actinin-positive rate was 99.4%.

iPS cells cultured in a 10 cm dish were washed with PBS (5 mL). Accutase (5 mL) was added, and the mixture was stood at 37° C under general oxygen conditions for 7 min. After 7 min, the cells were detached by pipetting, single celled, and subjected to centrifugation (1,000 rpm, 5 min). After centrifugation, the supernatant was removed, suspended in an undifferentiated maintenance medium added with Y-27632 10 µM, and seeded in an iMatrix-511 ((Nippi))-coated 96-well plate at a seeding density of 5×10³ cells/well.

The next day of seeding cardiomyocytes and single celled iPS cells, the medium was removed, and 100 µL of a fresh medium diluted with a compound (0.1 nM to 10 µM) or DMSO (0.1%) was added. The next day, the supernatant was removed, the cells were washed with PBS, and intracellular ATP was measured by an ATPlite 1 step ATP detection system (PerkinElmer). The results are shown in Fig. 2 (mean ± standard deviation, n=4). The effect of the treatment with compound on the intracellular ATP level was expressed as a relative value when the DMSO-treated cells of iPS cells and cardiomyocytes were taken as 100%.

From the results of the above-mentioned Experimental Examples 1 and 2, it was shown that the viable cell count of undifferentiated iPS cells remarkably decreases by the treatment with an HDAC inhibitor, but a decrease in the viable cell count is low in cardiomyocytes differentiated from iPS cells, irrespective of the kind of the iPS cell line.

### Experimental Example 3. Verification of effect of HDAC inhibitor in embryoid body containing mixture of cardiomyocytes and non-cardiomyocytes - 1

In view of the above-mentioned results, the present inventors considered that cardiomyocytes may be purified by using an HDAC inhibitor in an embryoid body with a mixture of cardiomyocytes and non-cardiomyocytes such as iPS cell and the like. Thus, the effect of an HDAC inhibitor on the embryoid body was verified by the following procedure.

### <iPS cell line>

A clinical iPS cell line produced by the CiRA was used. Maintenance culture of iPS cell line was performed according to the conventional method (Okita K, et al. Stem Cells. 2012 Nov 29. doi: 10.1002/stem.1293).

### <Induction of differentiation into cardiomyocytes>

Induction of differentiation into cardiomyocytes was performed according to the method described in <Induction of differentiation into cardiomyocytes> of the above-mentioned Experimental Example 1.

### <Treatment of embryoid body with compound, dissociation of embryoid body into single cells, and cell sorting>

On day 20, the embryoid body was recovered in a centrifugal tube, stood until a sediment of the embryoid body was formed, and the supernatant was aspirated while avoiding sucking the embryoid body. The medium was changed with a fresh I3 medium (same medium for myocardial differentiation as in Experimental Example 1), and divided into a 6-well plate with a medium amount of 3 mL/well so that an equal amount of the embryoid body would be placed in each well. Thereafter, an evaluation compound (1 nM to 10 µM) described in Table 1 or DMSO (0.1%) was added. The addition concentration of each compound (final concentration in the medium) is shown in Table 1. Thereafter, the cells were cultured at 37° C under general oxygen conditions for 3 days. Three days after the compound addition, the 6-well plate was stood to allow embryoid body to form sediments, and the embryoid body was recovered in a 1.5 mL tube by Pipetman with wide-diameter chips. It was centrifuged at 500 g for 1 min. The supernatant was removed, a solution of 500 µL of IMDM added with DNase 10 ug/mL and Liberase 100 µg/mL was added per one tube, and the mixture was stood at 37° C under general oxygen conditions for 1 hr. After 1 hr, the tube was centrifuged at 500 g for 1 min, and the supernatant was removed while avoiding sucking the embryoid body. Thereafter, a solution of 500 µL of TrypLE select added with DNase 10 µg/mL was added per tube, and the mixture was stood at 37° C under general oxygen conditions for 15 min. Thereafter, single cells were obtained by pipetting, 500 µL of IMDM medium added with DNase 10 µg/mL was added per tube, and the mixture was mixed by inversion and subjected to centrifugation (700 g, 5 min). After centrifugation, the supernatant was removed, and the cells were resuspended in Cytofix/Cytoperm Fixation/Permeabilization Solution (BD), and fixed by allowing to stand at room temperature for 15 min. The fixed cells were stained with an anti-sarcomeric α-actinin antibody, and the expression of sarcomeric α-actinin was analyzed with a flow cytometer. The results are shown in Fig. 3 (for DMSO-treated cells alone, mean ± standard deviation of n=3, cells treated with compound, n=1).

**[Table 1]**

| evaluation compound | addition concentration |
|---|---|
| Trichostatin A | 0.1 µM |
| | 0.2 µM |
| | 0.5 µM |
| | 1 µM |
| | 2 µM |
| Panobinostat | 0.1 µM |
| | 0.3 µM |
| | 1 µM |
| | 3 µM |
| FK-228 | 0.01 µM |
| | 0.03 µM |
| | 0.1 µM |

### Experimental Example 4. Verification of effect of HDAC inhibitor in embryoid body containing mixture of cardiomyocytes and non-cardiomyocytes - 2

Then, similar verification was performed using the same cell line as in Experimental Example 3, and it was confirmed that the cardiomyocyte rate was also improved with Entinostat, and that the cardiomyocyte rate was improved even with FK228 at a low concentration.

### <Induction of differentiation into cardiomyocytes>

Induction of differentiation into cardiomyocytes was performed according to the method described in <Induction of differentiation into cardiomyocytes> of the above-mentioned Experimental Example 1.

### <Treatment of embryoid body with compound, dissociation of embryoid body into single cells, and cell sorting>

On day 20, the embryoid body was recovered in a centrifugal tube, stood until a sediment of the embryoid body was formed, and the supernatant was aspirated while avoiding sucking the embryoid body. The medium was changed with a fresh I3 medium (same medium for myocardial differentiation as in Experimental Example 1), and divided into a 6-well plate with a medium amount of 3 mL/well so that an equal amount of the embryoid body would be placed in each well. Thereafter, an evaluation compound (1 nM to 10 µM) or DMSO (0.1%) was added. The concentration of each compound (final concentration in medium) is shown in Table 2. Thereafter, the cells were cultured at 37° C under general oxygen conditions for 4 days. Four days after the compound addition, the 6-well plate was stood to allow embryoid body to form sediments, and the embryoid body was recovered in a 1.5 mL tube by Pipetman with wide-diameter chips. It was centrifuged at 500 g for 1 min. The supernatant was removed, a solution of 500 µL of IMDM added with DNase 10 ug/mL and Liberase 100 µg/mL was added per one tube, and the mixture was stood at 37° C under general oxygen conditions for 1 hr. After 1 hr, the tube was centrifuged at 500 g for 1 min, and the supernatant was removed while avoiding sucking the embryoid body. Thereafter, a solution of 500 µL of TrypLE select added with DNase 10 µg/mL was added per tube, and the mixture was stood at 37° C under general oxygen conditions for 15 min. Thereafter, single cells were obtained by pipetting, 500 µL of IMDM medium added with DNase 10 µg/mL was added per tube, and the mixture was mixed by inversion and subjected to centrifugation (700 g, 5 min). After centrifugation, the supernatant was removed, and the cells were resuspended in Cytofix/Cytoperm Fixation/Permeabilization Solution (BD), and fixed by allowing to stand at room temperature for 15 min. The fixed cells were stained with an anti-sarcomeric α-actinin antibody, and the sarcomeric α-actinin-positive cell rate was measured by analyzing the expression of sarcomeric α-actinin with a flow cytometer. The results are shown in Fig. 4 (for DMSO-treated cells alone, mean (standard deviation) of n=3, cells treated with compound, n=1).

**[Table 2]**

| evaluation compound | addition concentration |
|---|---|
| FK-228 | 1 nM |
| | 2 nM |
| | 5 nM |
| | 10 nM |
| | 30 nM |
| Entinostat | 0.1 µM |
| | 1 µM |
| Trichostatin A | 10 µM |
| | 10 µM |

### Experimental Example 5. Verification of effect of HDAC inhibitor in embryoid body containing mixture of cardiomyocytes and non-cardiomyocytes - 3

Then, verification was performed using the same cell line as in Experimental Example 3, and it was confirmed that the non-cardiomyocyte rate decreased by the treatment with FK-228.

### <Induction of differentiation into cardiomyocytes>

Induction of differentiation into cardiomyocytes was performed according to the method described in <Induction of differentiation into cardiomyocytes> of the above-mentioned Experimental Example 1.

### <Treatment of embryoid body with compound, and dissociation of embryoid body into single cells>

On day 21, the embryoid body was recovered in a centrifugal tube, stood until a sediment of the embryoid body was formed, and the supernatant was aspirated while avoiding sucking the embryoid body. The medium was changed with a fresh I3 medium (same medium for myocardial differentiation as in Experimental Example 1), and divided into a 6-well plate with a medium amount of 3 mL/well so that an equal amount of the embryoid body would be placed in each well, and FK-228 (0.1 nM, 1 nM) or DMSO (0.1%) was added. Thereafter, the cells were cultured at 37° C under general oxygen conditions for 3 days. Three days after the compound addition, the 6-well plate was stood to allow embryoid body to form sediments, and the embryoid body was recovered in a 1.5 mL tube by Pipetman with wide-diameter chips. It was centrifuged at 500 g for 1 min. The supernatant was removed, a solution (3 mL) obtained by adding DNase 10 µg/mL and Liberase 100 µg/mL to 1 mL of IMDM (Iscove's Modified Dulbecco's Media) was added per one tube, and the mixture was stood at 37° C under general oxygen conditions for 1 hr. After 1 hr, the tube was centrifuged at 500 g for 1 min, and the supernatant was removed while avoiding sucking the embryoid body. A solution (500 µL) obtained by adding DNase 10 µg/mL to TrypLE select (Thermo) was added to each tube, and the mixture was stood at 37° C under general oxygen conditions for 10 min. After standing, single cells were obtained by pipetting, a medium (500 µL) obtained by adding DNase 10 µg/mL to IMDM was added to each tube, and the mixture was mixed by inversion to prepare a single cell suspension.

The above-mentioned single cell suspension was centrifuged at 400 g for 3 min, the supernatant was removed, and the cells were suspended in Bambanker and cryopreserved at -80° C. The cryopreserved cells were thawed in a warm bath at 37° C and centrifuged at 400 g for 5 min, and the supernatant was removed. After tapping the cell pellets, PBS containing 1% BSA was added, and the mixture was centrifuged at 300 g for 1 min. The supernatant was removed. The cells were stained with APCFire750-labeled anti-CD326 antibody, PE-labeled anti-CD49a antibody, BV605-labeled anti-CD31 antibody, and DAPI in PBS containing 1% BSA. DAPI-positive dead cells were removed, and the separation of cell population and the ratio of each cell population were measured by measuring the signal amount of each fluorescence dye in cell.

Cardiomyocytes obtained by differentiation induction of the iPS cell line into cardiac muscle were separated into 4 main cell populations of CD326-positive cells (endoderm lineage cells), CD326-negative CD31-positive cells (vascular endothelium-like cells), CD326-negative CD31-negative CD49a-positive cells (smooth muscle-like cells), and CD326-negative CD31-negative CD49a-negative cells based on the differences in the expression of these three surface markers.

The results are shown in Fig. 5 (for DMSO-treated cells alone, mean (standard deviation) of n=3, cells treated with compound, n=1).

From the above, it was shown that cardiomyocytes in embryoid bodies can be purified by HDAC inhibition.

### [Industrial Applicability]

According to the present invention, a cell population containing cardiomyocytes at a high purity is provided. Such cell population is useful because it can be preferably used for cell transplantation therapy for cardiac diseases and screening for therapeutic agents for cardiac diseases.

This application is based on a patent application No. 2020-050269 (filing date: March 19, 2020) and a patent application No. 2020-145097 (filing date: August 28, 2020) filed in Japan, the contents of which are incorporated in full herein.

## Claims

1. A method for producing a cell population comprising cardiomyocytes, comprising
(1) a step of bringing a histone deacetylase inhibitor into contact with a cell population containing cardiomyocytes and cells other than cardiomyocytes, the cell population being obtained by culturing pluripotent stem cells in a medium for cardiomyocyte differentiation, and
(2) a step of culturing the cell population.

2. The method according to claim 1, wherein the cell population is contacted with the histone deacetylase inhibitor in the step (1) on or after 7 days from the start of differentiation induction of pluripotent stem cells.

3. The method according to claim 1 or 2, wherein the inhibitor is an inhibitor against class I histone deacetylase.

4. The method according to any one of claims 1 to 3, wherein the inhibitor is at least one member selected from the group consisting of FK228, Entinostat, Trichostatin A, and Panobinostat.

5. The method according to any one of claims 1 to 4, wherein the pluripotent stem cell is an induced pluripotent stem cell.

6. A cell populationcomprising cardiomyocytes and obtained by the method according to any one of claims 1 to 5.

7. An agent for cell transplantation therapy, comprising the cell population according to claim 6.

8. A method for purifying cardiomyocytes, comprising
(1) a step of bringing a histone deacetylase inhibitor into contact with a cell population containing cardiomyocytes and cells other than cardiomyocytes, the cell population being obtained by culturing pluripotent stem cells in a medium for cardiomyocyte differentiation, and
(2) a step of culturing the cell population.
